# EUROPEAN PATENT APPLICATION

(11) **EP 0 800 797 A1**
(43) Date of publication of application: **15.10.1997**
(21) Application number: 97830162.0
(22) Date of filing: 04.04.1997
(51) Int. Cl.: A61B 19/02

(54) **Container made of sterile wadding**

(30) Priority: 11.04.1996 IT OR960001 U
(71) Applicant: Dessi' Lai, Gianfranco, 09072 Cabras OR (IT)
(72) Inventor: Dessi' Lai, Gianfranco, 09072 Cabras OR (IT)

(57) **Abstract**

contaniner made of sterile wadding to be used during surgical operations. It's composed of different receptacles where cotton tampons and ganzes can be deposited and kept as a store.
Many times the news have reported of deceases due to the forgetfulness of foreing bodies inside the human body. The praticalness of this the containers makes possible the 15 count of what is engaged and recovered. The surgical operation can be considered finished only after a correct verification.

## Description

Tecnical field: contaniner made of sterile wadding to be used during surgical operations. It's composed of different receptacles where cotton tampons and ganzes can be deposited and kept as a store.
Sterilization happens through autoclave.

Background art: at present containers made of wadding aren't used during surgical operations. The ones used are metallic cans which are not separated in sectors.

Disclosure of invention. Advantages: Many times the news have reported of deceases due to the forgetfulness of foreing bodies inside the human body. The praticalness of this the containers makes possible the count of what is engaged and recovered. The surgical operation can be considered finished only after a correct verification.

Mode for carryng out the invention: The contaniners I conceived are similar to those used to keep eggs in but the material, the dimensions and the shape are different.
Therefore the machines constructives will be adapted to such characteristics. The number of the receptacles and the dimension of the containers are adapted on request.

The praticalness of this container compared with the metallic can is that it will be eliminated at the end of the operation.
Lastly I'd be very glad if the Ministery of Health allows the use of these containers, so that the above quoted incidents can be obviated.

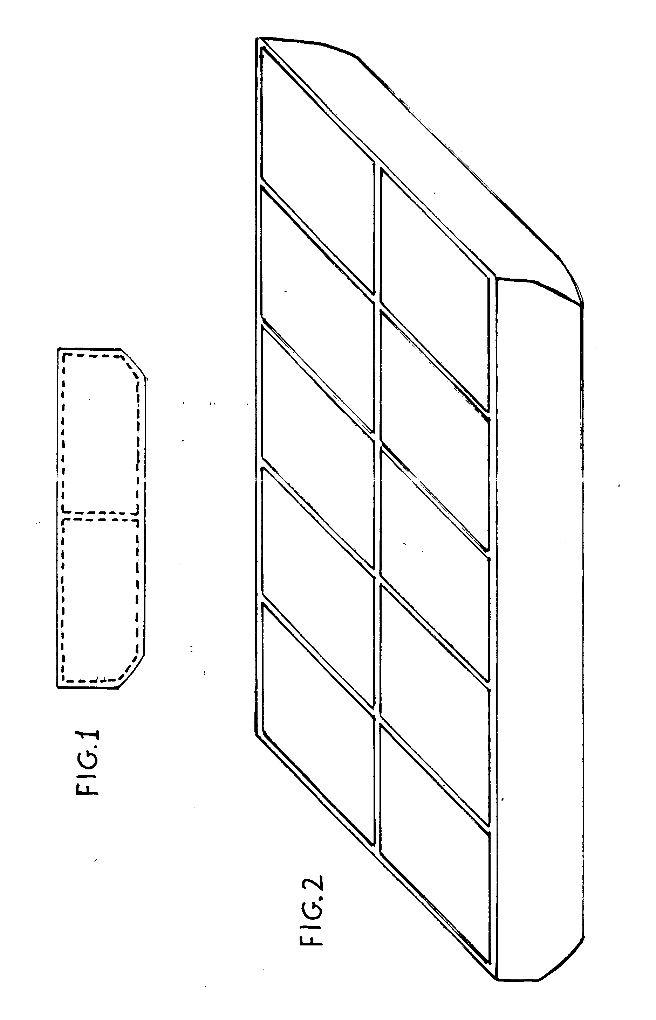

## Claims

1. At present containers made of wadding aren't used during surgical operations. The ones used are metallic cans which are not separated in sectors.
In case of need the wadding is biodegradable or incinerable.
On request the container is produced with cotton tampons, ganzes or differents accessories into the receptacles and covered with a film of cellofan.
